# DEMANDE DE BREVET EUROPEEN

(11) **EP 4 481 107 A1**
(43) Date de publication de la demande: **25.12.2024**
(21) Numéro de dépôt: 24183484.5
(22) Date de dépôt: 20.06.2024
(51) Int. Cl.: D06N 3/00, D06N 3/04, D06N 3/10, D06N 3/12, D06N 3/14, D06N 3/18, B32B 27/12, A61N 1/14, A61N 5/06

(54) **ARTICLE TEXTILE POUR MISE À LA TERRE AVEC RENVOI DE RAYONS INFRAROUGE**

(30) Priorité: 21.06.2023 FR 2306416
(71) Demandeur: Nouvelle Terre, 26220 Dieulefit (FR)
(72) Inventeur: TORTEL, Arnaud, 26220 DIEULEFIT (FR)
(74) Mandataire: Germain Maureau

(57) **Abrégé**

L'invention concerne un article textile (1a) configuré pour être mis à la terre comprenant :
- une couche d'un matériau textile (2a) présentant une 1^{ère} face (3) et une 2^{ème} face (4),
- un système de mise à la terre (10) de l'article textile (1a),
et qui se caractérise en ce que :
- la 1^{ère} face (3) de la couche de matériau textile (2a) est recouverte d'un 1^{er} dépôt (5) comprenant un polymère et des particules de matériau conducteur (noir de carbone),
- la 2^{ème} face (4) de la couche de matériau textile (2a) est recouverte d'un 2^{ème} dépôt (6) comprenant un polymère et des oxydes métalliques.

## Description

La présente invention concerne un article textile configuré pour être mis à la terre. Il peut s'agir notamment d'une natte de lit, de surmatelas, d'oreiller, de couette, d'une assise pour siège de bureau ou de voiture, d'une couverture pour équidés ou encore d'un vêtement tel qu'un T-shirt, d'un pull, d'une veste, d'une doudoune, d'un pantalon, de chaussettes, ou encore de semelle.

La surface de la terre est chargée négativement et véhicule en permanence des électrons. Ainsi, lorsque le corps humain se « connecte » à la terre en étant pieds nus, les électrons libres à la surface de la terre sont transférés au corps.

D'un point de vue métabolique les radicaux libres, les déchets cellulaires et les oxydations corporelles sont chargés positivement et ont besoin d'avoir des apports en électrons pour être éliminés. En effet, les inflammations, les radicaux libres, mais aussi les fractures, les déchirures, les courbatures et les douleurs se développent en milieu acide chargé positivement.

C'est pourquoi, la connexion à la terre par les pieds nus (ou éventuellement recouverts de cuir) apporte naturellement des électrons antioxydants nécessaires. La Terre étant un « énorme » condensateur, elle permet d'absorber et de réguler les flux d'électrons du corps afin de réduire les stases oxydatives et de piéger les radicaux libres, principales sources d'inflammations aigues et chroniques. Ainsi, les carences en électrons et les excès de radicaux libres et d'acidité dans le corps sont en partie corrigés.

En entrant en contact direct avec la terre, nous permettons à notre corps d'échanger des électrons. Ce phénomène dit de « mise à la terre », aussi connu sous les dénominations anglophones d'« earthing » et de « grounding », permet ainsi d'obtenir des effets bénéfiques sur la santé humaine. A cet égard, plusieurs études scientifiques détaillent ces effets bénéfiques. Il s'agit notamment d'une réduction de l'inflammation, d'une augmentation de l'endurance, une régulation du stress, une régulation de la circulation sanguine et d'une amélioration du sommeil.

Cependant, depuis quelques dizaines d'années avec l'avènement des semelles en plastiques, des revêtements en bois ou stratifiés, du goudron et autres revêtements isolants, ce contact direct avec la terre permettant à notre corps d'échanger des électrons est rompu, et par voie de conséquence ses bienfaits sur la santé. De plus, nous gardons des charges positives sur le corps qui finissent par créer des effets d'électricité statique entre nos vêtements.

En résumé, la Terre fournit la nutrition électrique du sol sous nos pieds pour maintenir l'ordre naturel dans les circuits bioélectriques du corps. De plus, le rythme quotidien du cycle chimique et hormonal dans le corps humain est en partie régulé et maintenu par de faibles signaux électromagnétiques générés par les électrons de la Terre. Le corps humain est extrêmement réceptif à ces signaux qui établissent le rythme des fonctions jour-nuit ou des rythmes circadiens. Nous avons un réel besoin des électrons de la Terre pour notre bien-être. Ils sont fondamentaux pour la santé et leur déficience peut causer des problèmes de santé.

Par ailleurs, ces 100 dernières années ont vu l'apparition généralisée de l'électricité, des ondes radios, des radars, des micro-ondes, de l'accès sans fil à l'internet, ainsi que de la téléphonie. Nous sommes entourés et « bombardés » en permanence d'ondes électromagnétiques de fréquences différentes que les scientifiques nomment aujourd'hui le "brouillard électromagnétique". En suivant les lois de la physique des ondes, nous pouvons déterminer l'impact de chaque type d'ondes magnétiques ou électriques sur les cellules humaines. Toutes ces ondes ont tendance à perturber les échanges cellulaires et à favoriser les processus inflammatoires, oxydatifs et les radicaux libres. Ce brouillard électromagnétique génère un terrain inflammatoire chronique à l'origine notamment de maladies, de troubles musculo-squelettiques, de manque d'attention et de fatigue chronique. Nous avons donc aussi un besoin accru d'électrons pour piéger toutes les nombreuses toxines créées par ce brouillard électromagnétique.

C'est pourquoi, retrouver une connexion à la terre, la meilleure possible, est devenue nécessaire pour piéger l'ensemble des phénomènes d'oxydation engendrés, notamment par les ondes électriques et magnétiques de notre environnement. Cela est d'autant plus vrai pour les personnes travaillant dans des bureaux devant des ordinateurs. A cet égard, les normes européennes en matière d'électricité induite dans le corps humain préconisent de ne pas dépasser 3 à 5 V/m. Or, il est fréquent que nous mesurions dans les bureaux des taux allant de 5 à 80 V/m devant un ordinateur branché sur secteur.

Il existe des dispositifs de mise à la terre. Il peut s'agir de tapis, de drap, d'assise pour siège de bureau ou de voiture qui d'une part, sont configurés pour être mis à la terre et d'autre part, comprennent une partie textile conductrice (par exemple à base de fibres de carbone ou de fibres de nylon recouvertes d'argent). Dès lors que le dispositif est mis à la terre et qu'une partie du corps est en contact direct avec la partie textile conductrice, une connexion à la terre est établie ; ce qui permet l'échange d'électrons avec le corps expliqué ci-dessus, ainsi que les bienfaits sur la santé également détaillés ci-dessus. A cet égard, la demande US 2008/0068773 A1 et le brevet US 6,683,779 B2 décrivent des exemples de dispositifs de mise à la terre.

Par ailleurs, au cours des dernières décennies, un nombre croissant de recherches ont porté sur l'utilisation du rayonnement infrarouge (ci-après abrégé « IR ») pour des effets bénéfiques sur la santé. L'IR est un rayonnement électromagnétique, non ionisant, dont la longueur d'onde est comprise entre environ 750 nm et 1 mm. Les rayons IR proviennent de l'énergie thermique. Ainsi, les corps matériels émettant de la chaleur peuvent les produire. Par conséquent, le corps humains est un émetteur de rayonnement IR.

D'un point de vue biologique, les rayons IR peuvent affecter les tissus vivants du niveau cellulaire au niveau systémique. Au niveau moléculaire, les rayons IR affectent la rotation des molécules contenues dans les fluides corporels et les tissus. L'effet clinique et physiologique potentiel dépend de la composition du tissu et de la proportion de biomolécules contenues dans les liquides fluides corporels. Au niveau cellulaire, l'interaction entre les rayons IR et les tissus vivants se résume à des altérations des potentiels de la membrane cellulaire induites par la chaleur, par le biais d'une augmentation des niveaux de Ca²⁺ intracellulaires. Au niveau tissulaire, une augmentation des niveaux d'oxyde nitrique, une substance antioxydante endogène contrant la production des dérivés réactifs de l'oxygène, est associée à la réduction du stress oxydatif et à la vasodilatation. Simultanément, il stimule la production de facteurs de croissance et le dépôt de matrice extracellulaire, ce qui favorise la réparation des tissus. En ce sens, l'amélioration de la circulation sanguine associée aux rayons IR peut favoriser la guérison des blessures et des escarres, diminuer les spasmes musculaires et améliorer la vitesse de conduction nerveuse sensorielle, et potentiellement augmenter les endorphines qui modulent la douleur.

Les nombreuses actions potentielles du rayonnement IR ont fait l'objet de diverses études. En résumé, les avantages sur la santé des rayons IR sont les suivants :
- une amélioration de la circulation sanguine : les rayons IR augmentent le flux sanguin et la circulation, ce qui contribuer à améliorer la santé et le bien-être en général ;
- un soulagement de la douleur : les rayons IR sont efficaces pour réduire la douleur et l'inconfort dans diverses conditions, telles que les douleurs musculaires et l'arthrite ;
- une réduction de l'inflammation : les rayons IR ont des propriétés anti-inflammatoires, ce qui contribue à réduire les gonflements et les inflammations dans le corps ;
- un renforcement du système immunitaire : les rayons IR contribuent à renforcer le système immunitaire ; ce qui aide à se protéger contre les maladies ;
- une amélioration de la santé cardiovasculaire : les rayons IR contribuent à améliorer la santé cardiaque en réduisant la pression artérielle, en abaissant le taux de cholestérol et en réduisant le risque de maladie cardiaque ;
- une aide pour la perte de poids : les rayons IR favorisent la perte de poids en augmentant le taux métabolique du corps et en brûlant des calories ;
- une amélioration du sommeil.

Il existe des dispositifs (par exemple des tapis ou des nattes de lit) qui comportent d'une part des éléments pour une mise à la terre du corps humain et d'autre part des éléments configurés pour renvoyer des rayons IR émis par le corps humain. De tels dispositifs combinent ainsi à la fois les effets bénéfiques sur la santé de la mise à la terre et de ceux procurés par le renvoi des rayons IR qui ont été détaillés ci-dessus.

A cet égard, la demande KR10-2019-0119256 A décrit un exemple d'un tel dispositif qui se présente sous la forme d'un tapis comportant un empilement de couches de différents matériaux parmi lesquelles figurent :
- une couche en céramique pour l'émission de rayons IR,
- une couche réalisée en un matériau conducteur et qui est reliée par un fil de connexion conducteur à la terre.

Le tapis faisant l'objet d'un exemple de mode de réalisation de cette demande de brevet coréenne comporte 9 couches de matériaux différents. La structure de ce tapis configuré pour être mis à la terre est particulièrement complexe et a donc nécessité l'assemblage de 9 couches distinctes ; ce qui rend son procédé de fabrication compliqué, onéreux, nécessitant beaucoup de matériaux différents et ainsi peu attractif.

Le brevet chinois CN 105 040 234 B décrit une natte qui comprend :
- un tissu conducteur comprenant un élément de connexion à un système de mise à la terre ;
- un non tissé ;
- un tissu fonctionnel qui a été obtenu à partir de coton synthétique et d'un mélange fondu de résine synthétique (polyester) et de poudre de tourmaline.

Le tissu conducteur comprend des fils d'argent et des fils de polyester ou de coton. Le tissu fonctionnel émet des rayons IR. La tourmaline a été intégrée en tant qu'additif au moment de la fabrication du fil en coton synthétique. Elle est donc répartie dans tout le fil et non pas qu'en surface dudit fil.

La natte décrite dans le brevet chinois CN 105 040 234 B comprend ainsi trois matériaux textiles différents qui sont superposés les uns sur les autres, donc une structure complexe avec différents matériaux textiles.

L'inventeur de la présente invention a cherché à surmonter notamment les inconvénients quant aux structures complexe du tapis mis à la terre faisant l'objet de la demande de brevet coréen précitée et de la natte décrite dans le brevet chinois précité et a mis au point un article textile configuré pour être mis à la terre qui présentent les avantages suivants :
- une structure simple, légère et parfaitement fiable et bénéfique pour la santé ;
- un procédé de fabrication simple et aisé à mettre en oeuvre, peu onéreux, nécessitant un minimum de matières.

L'invention a ainsi pour premier objet un article textile configuré pour être mis à la terre qui comprend au moins :
- une couche d'un matériau textile présentant une 1^{ère} face et une 2^{ème} face,
- un système de mise à la terre de l'article textile,
ledit article textile se caractérise en ce que :
- la 1^{ère} face de la couche de matériau textile est totalement ou en partie recouverte d'un 1^{er} dépôt comprenant au moins un polymère et des particules d'au moins un matériau conducteur, ledit 1^{er} dépôt étant optionnellement en contact avec le système de mise à la terre,
- la 2^{ème} face de la couche de matériau textile est totalement ou en partie recouverte d'un 2^{ème} dépôt comprenant au moins un polymère et des oxydes métalliques.

Du fait que le 1^{er} dépôt comprend des particules d'au moins un matériau conducteur, ce 1^{er} dépôt permet le passage des électrons de manière continue sur l'article textile selon l'invention lorsque celui-ci est mis à la terre (autrement dit branché à la terre) avec le système de mise à la terre. Cela permet de reproduire le phénomène naturel de décharge électrostatique.

Du fait que le 2^{ème} dépôt comprend des oxydes métalliques, ce 2^{ème} dépôt permet le renvoi des rayons IR émis par le corps humain, et ce de préférence à plus de 90% dans le but d'obtenir les bienfaits sur la santé qui ont été rappelés ci-dessus (à savoir notamment l'augmentation de la microcirculation durant la nuit, favorisant la détente, l'oxygénation des tissus et l'élimination des déchets).

Ainsi, lorsque l'article textile selon l'invention est mis à la terre, il suffit qu'une partie du corps soit en contact avec le 1^{er} dépôt pour recevoir tous les bienfaits dudit article textile. Plus précisément, grâce au 1^{er} dépôt, les électrons remontent et parcourent la surface du corps. Dans le même temps, grâce au 2^{ème} dépôt, le renvoi de rayons IR émis par le corps stimule la microcirculation des muscles et des organes. De plus, du fait de ce 2^{ème} dépôt, les électrons pénètrent beaucoup plus profondément sous le derme afin de venir directement au coeur des zones d'oxydation.

Les effets bénéfiques sur la santé avec l'article textile selon l'invention sont notamment les suivants :
- une amélioration de l'oxygénation du sang,
- un calme des troubles digestifs et des ballonnements,
- une régulation du cycle hormonal,
- une réduction de l'ostéoporose et une amélioration du métabolisme calcique,
- une réduction des inflammations articulaires et une diminution des douleurs liées à l'arthrite,
- une réduction des crampes et courbatures liées à l'effort,
- une équilibration du système ortho-parasympathique,
- une réduction du stress,
- une amélioration du sommeil et une augmentation du sommeil profond,
- une augmentation de la variabilité de la fréquence cardiaque (ci-après abrégée « VFC ») par stimulation du système parasympathique,
- une accalmie des arythmies et diminution de la pression sanguine,
- une amélioration de la réponse immunitaire et une accélération de la cicatrisation,
- une diminution des effets du stress oxydatif (radicaux libres),
- une prévention et une réduction des inflammations chroniques,
- une meilleure récupération.

De préférence, la 1^{ére} face de la couche de matériau textile est totalement recouverte du 1^{er} dépôt.

De préférence, la 2^{ème} face de la couche de matériau textile est totalement recouverte du 2^{ème} dépôt.

L'épaisseur du 1^{er} dépôt peut être comprise entre 20 µm et 2 mm, de préférence entre 100 µm et 1 mm.

L'épaisseur du 2^{ème} dépôt peut être comprise entre 20 µm et 2 mm, de préférence entre 100 µm et 1 mm.

Les 1^{er} et 2^{ème} dépôts sont d'épaisseur très fine et ajoutent ainsi très peu d'épaisseur à l'article textile. En d'autres termes, l'épaisseur de l'article textile selon l'invention est essentiellement constituée par l'épaisseur de la couche de matériau textile.

A la différence du dispositif de mise à la terre décrit dans la demande de brevet coréen mentionnée ci-dessus, l'article textile selon l'invention présente l'avantage d'être de très faible épaisseur et peut donc s'adapter sur tous types de support, par exemple un matelas, un siège de bureau ou d'automobile.

En d'autres termes, dans l'article textile selon l'invention, la 1^{ère} face de la couche de matériau textile a été enduite d'un 1^{er} dépôt comprenant au moins un polymère et des particules d'au moins un matériau conducteur et la 2^{ème} face de la couche de matériau textile a été enduite d'un 2^{ème} dépôt comprenant au moins un polymère et des oxydes métalliques.

A la différence des dispositifs de mise à la terre de l'art antérieur, l'article textile selon l'invention est de structure très simple puisqu'il s'agit d'une couche de matériau textile qui a été enduite sur chacune de ses faces de respectivement un 1^{er} dépôt et un 2^{ème} dépôt. Il ne s'agit donc pas d'une superposition de différents matériaux textiles comme cela a été décrit dans l'art antérieur.

Le matériau textile peut être choisi parmi le coton, le polyamide, le polyester, la viscose, le polypropylène, des matériaux ignifugés, la laine et la soie. De préférence, le matériau textile est du coton ou du polyamide ou du polyester.

Par exemple, il peut s'agir d'un tissu résille, notamment en polyester, créant l'effet de micro-perforations. C'est pourquoi, il est dénommé ci-après « polyester micro-perforé ». Son épaisseur peut être par exemple de 2 mm et son grammage de 80 à 300 g/m², de préférence de 110 à 180 g/m².

Le matériau textile peut être choisi parmi les matériaux textile non-tissés, tissés, tricotés, ainsi que les matériaux textiles de rembourrage (par exemple ouate ou mousse de polyuréthane).

L'épaisseur de la couche de matériau textile peut être comprise entre 1 mm et 5 cm.

Lorsque le matériau textile est un matériau de rembourrage, l'épaisseur dudit matériau textile peut être comprise entre 5 mm et 5 cm.

Lorsque le matériau textile n'est pas un matériau de rembourrage, l'épaisseur dudit matériau textile peut être comprise entre 1 mm et 2 cm, de préférence entre 1 mm et 10 mm.

La couche de matériau textile peut comprendre une seule couche de matériau textile.

Dans d'autres modes de réalisation de l'invention, la couche de matériau textile est composée de plusieurs couches de matériau textile qui sont superposées les unes sur les autres. Les matériaux textiles de ces couches de matériau textile peuvent être choisis parmi ceux décrits ci-dessus.

Dans ces modes de réalisation de l'invention :
- la 1^{ère} face de la couche de matériau textile correspond à la face de la couche de matériau textile dite « couche supérieure » qui n'est pas en contact avec la couche de matériau textile précédente sur laquelle est disposée ladite couche supérieure,
- la 2^{ème} face de la couche de matériau textile correspond à la face de la couche de matériau textile dite « couche inférieure » qui n'est pas en contact avec la couche de matériau textile suivante qui est disposée sur ladite couche inférieure.

En d'autres termes, la couche inférieure est la 1^{ère} couche de matériau textile sur laquelle sont superposées les unes sur les autres les couches suivantes de matériau textile. La couche supérieure étant la dernière couche de matériau textile de cet empilement de couches de matériau textile.

Lorsque la couche de matériau textile comprend deux couches, à savoir une couche inférieure sur laquelle est disposée une couche supérieure :
- la 1^{ère} face de la couche de matériau textile correspond à la face de la couche supérieure qui n'est pas en contact avec la couche inférieure,
- la 2^{ème} face de la couche de matériau textile correspond à la face de la couche inférieure qui n'est pas en contact avec la couche supérieure.

Des exemples de couche de matériau textile composée de plusieurs couches de matériau textile sont décrits sans que cela n'en limite pour autant la portée de la présente invention.

La couche de matériau textile peut être une couche de coton de grammage 140 g/m² ou de polyester micro-perforé de grammage 180 g/m² par exemple. Ce type de couche de matériau textile est particulièrement approprié lorsque l'article textile selon l'invention est une natte ou une couverture pour équidés.

La couche de matériau textile peut aussi se composer d'une 1^{ère} couche de matériau textile de polyester (par exemple avec un grammage de 110 g/m²) sur laquelle est disposée une 2^{ème} couche de matériau textile de rembourrage comprenant de la ouate. Une 3^{ème} couche de matériau textile de polyester (par exemple avec un grammage de 110 g/m²) est disposée sur ladite 2^{ème} couche de matériau textile. La couche de matériau textile comprend alors 3 couches de matériau textile. Selon l'épaisseur de la couche de rembourrage et la quantité d'ouate, ce type de couche de matériau textile est particulièrement approprié lorsque l'article textile selon l'invention est une natte matelassée, un matelas, un coussin, une veste doudoune ou encore une couverture hivernale matelassée pour équidés.

La couche de matériau textile peut aussi se composer d'une 1^{ère} couche de polyester (par exemple avec un grammage de 110 g/m²) sur laquelle est disposée une 2^{ème} couche de matériau textile de rembourrage comprenant une mousse à mémoire de forme, par exemple une mousse de polyuréthane. Une 3^{ème} couche de matériau textile de polyester (par exemple avec un grammage de 110 g/m²) est disposée sur ladite 2^{ème} couche de matériau textile. La couche de matériau textile comprend alors 3 couches de matériau textile. Selon l'épaisseur de la couche de rembourrage, ce type de couche de matériau textile est particulièrement approprié lorsque l'article textile selon l'invention est un matelas à mémoire de forme, un coussin à mémoire de forme ou encore une couverture hivernale matelassée pour chevaux.

Le polymère du 1^{er} dépôt peut être choisi dans le groupe constitué par les acryliques, les polyuréthanes et les silicones, pris seul ou en combinaison de ceux-ci. De préférence, il s'agit d'acryliques ou de polyuréthane.

Le matériau conducteur des particules du 1^{er} dépôt peut être choisi parmi le carbone (par exemple noir de carbone), l'argent, le nickel, le cuivre, l'acier inoxydable, le zinc et le cobalt, pris seul ou en mélange de ceux-ci.

La taille des particules du 1^{er} dépôt peut être comprise entre 20 nanomètres et 20 micromètres, de préférence entre 0,5 et 5 microns.

De manière tout à fait préférée, les particules du 1^{er} dépôt sont du noir de carbone ou noir d'acétylène (aussi connu sous la dénomination de noir de carbone d'acétylène).

Le 1^{er} dépôt peut comprendre, en pourcentages massiques exprimés par rapport à la masse du 1^{er} dépôt :
- entre 1% et 10% de particules de matériau conducteur, de préférence de noir de carbone,
- entre 90% et 99% de polymère, de préférence au moins un polymère choisi parmi ceux décrits ci-dessus.

Le 1^{er} dépôt peut comprendre au moins 1 g/m², de préférence entre 20 g/m² et 150 g/m², de particules de matériau conducteur, de préférence du noir de carbone.

Du fait que la 1^{ère} face de la couche de matériau textile a été enduite d'un 1^{er} dépôt tel que décrit ci-dessus, l'article textile selon l'invention présente l'avantage de présenter un grammage en matériau conducteur plus important que celui de dispositifs de mise à la terre connus de l'art antérieur qui comportent un tissu tissé à partir de fils de matériau conducteur (par exemple des fils d'argent ou de carbone) ou de fils enduits en surface avec des particules de matériau conducteur.

Ainsi, du fait d'un grammage en matériau conducteur plus élevé, les bienfaits procurés par ledit matériau conducteur sont plus importants avec l'article textile selon l'invention.

Le polymère du 2^{ème} dépôt peut être choisi dans le groupe constitué par les acryliques, les polyuréthanes, les copolymères polystyrène/butadiène, le latex naturel ou synthétique, le gel d'élastomère de silicone, l'élastomère de silicone, le caoutchouc butyle, le caoutchouc naturel, le caoutchouc nitrile, le styrène bloc copolymère et le styrène, pris seul ou en combinaison de ceux-ci. De préférence, il s'agit d'acrylique ou de polyuréthane.

Les oxydes métalliques du 2^{ème} dépôt sont avantageusement choisis parmi les oxydes métalliques capables de renvoyer les rayons IR dans l'intervalle de longueurs d'onde compris entre 3 et 20 µm.

Les oxydes métalliques du 2^{ème} dépôt peuvent être choisis dans le groupe constitué par les oxydes d'aluminium, de titane, de zirconium, d'yttrium, de magnésium, de silicium, de fer, de cobalt, de manganèse, de cuivre, de zinc et de chrome, pris seul ou en combinaison de ceux-ci. De préférence, il s'agit d'un mélange de tous les oxydes précités.

La taille de l'oxyde métallique peut être comprise entre 0,2 µm et 20 µm.

Le 2^{ème} dépôt peut en outre comprendre un solvant choisi parmi l'eau, l'acétate d'éthyle, l'acétate de butyle et le méthyl éthyl cétone.

Le 2^{ème} dépôt peut en outre comprendre un composé hydrophile choisi parmi les carboxy-méthyl-celluloses et le polyéthylène glycol.

Le 2^{ème} dépôt peut en outre comprendre un composé choisi parmi des agents dispersants, réticulants ou fixateurs.

Le 2^{ème} dépôt peut comprendre, en pourcentages massiques exprimés par rapport à la masse du 2^{ème} dépôt :
- entre 5% et 50% d'oxydes métalliques,
- entre 50% et 95% de polymère, de préférence au moins un polymère choisi parmi ceux décrits ci-dessus,
- optionnellement entre 5% et 80%, de préférence entre 5% et 20%, de solvant,
- optionnellement entre 2% et 20% d'un composé hydrophile choisi parmi les carboxy-méthyl-celluloses et le polyéthylène glycol,
- optionnellement entre 1% et 10% de composés additionnels, de préférence au moins un composé additionnel choisi parmi ceux décrits ci-dessus.

Le 2^{ème} dépôt comprend entre 5 g/m² et 150 g/m² d'oxydes métalliques.

Du fait que la 2^{ème} face de la couche de matériau textile a été enduite d'un 2^{ème} dépôt tel que décrit ci-dessus, l'article textile selon l'invention présente l'avantage de présenter un grammage en oxydes métalliques plus important que celui de dispositifs connus de l'art antérieur qui comportent un tissu tissé à partir de fils enduits en surface avec des oxydes métalliques ou bien dont les oxydes métalliques ont été incorporés en tant qu'additifs lors de la fabrication dudit fil de telle sorte que les oxydes métalliques soient répartis dans tout le fil et non pas qu'en surface.

Ainsi, du fait dans un article textile selon l'invention le grammage en oxydes métalliques est 10 à 50 fois plus élevé que celui d'articles textiles de l'art antérieur dans lesquels les oxydes métalliques sont induits sur des fils ou introduits dans la composition des fils, les bienfaits procurés par les oxydes métalliques sont plus importants avec l'article textile selon l'invention.

Dans un mode de réalisation avantageux de l'invention, un tissu résistant à la déchirure peut être fixé (par exemple par collage ou par couture) sur une partie ou sur la totalité du 2^{ème} dépôt. Ce tissu résistant à la déchirure peut par exemple être un tissu synthétique, réalisé notamment en polyester ou en polyamide. Un exemple de tissu résistant à la déchirure est par exemple le tissu commercialisé sous la dénomination commerciale « RIPSTOP ^{®} ». Ce tissu résistant à la déchirure permet de renforcer la structure de l'article textile selon l'invention et donc d'augmenter sa solidité.

Dans un autre mode de réalisation avantageux de l'invention, un tissu résille (par exemple un tissu résille en polyester) peut être fixé par couture ou par collage sur une partie ou sur la totalité du 2^{ème} dépôt. Ce mode de réalisation de l'invention est particulièrement approprié lorsque l'article textile est une natte. Ce tissu permet de mieux fixer la natte sur le lit en évitant qu'elle se plisse et qu'elle se déplace sous le drap housse sous l'action des mouvements du dormeur.

Dans un autre mode de réalisation avantageux de l'invention, un tissu résille (par exemple un tissu résille en polyester) peut être fixé par couture ou par collage sur une partie ou sur la totalité du 2^{ème} dépôt et un tissu résistant à la déchirure (par exemple un tissu tel que décrit ci-dessus) peut être fixé sur ce tissu résille. Ce mode de réalisation de l'invention est particulièrement approprié lorsque l'article textile est une couverture pour équidés.

Dans un autre mode de réalisation avantageux de l'invention, des éléments en silicone sont fixés sur le 2^{ème} dépôt, le cas échéant sur le tissu résille ou le tissu résistant à la déchirure, de manière à augmenter la stabilité de l'article textile selon l'invention. De cette manière, une fois mis en place à l'endroit souhaité (par exemple sur le matelas), l'article textile selon l'invention ne risquera pas d'être déplacé de manière intempestive, et ce afin de garantir tous les bienfaits de cet article textile lors de sa mise à la terre.

Dans un autre mode de réalisation avantageux de l'invention, un tissu respirant à larges pores (par exemple en polyester, polyamide, coton, viscose, polypropylène peut être fixé par couture ou par collage sur une partie ou sur la totalité du 2^{ème} dépôt. Ce mode de réalisation de l'invention est particulièrement approprié lorsque l'article textile est une veste doudoune.

Dans des modes de réalisation de l'invention, le système de mise à la terre de l'article textile selon l'invention peut comprendre :
- un fil de connexion en matériau conducteur (par exemple du cuivre) présentant une 1^{ère} extrémité configurée pour être connectée sur le 1^{er} dépôt et une 2^{ème} extrémité configurée pour être connectée à un moyen de mise à la terre.

Plus précisément, le 1^{er} dépôt peut comprendre un élément de connexion à la 1^{ère} extrémité du fil de connexion. Il peut par exemple s'agir d'un rivet et la 1^{ère} extrémité du fil de connexion peut comprendre une pince crocodile. De cette manière, la 1^{ère} extrémité du fil de connexion est connectée au 1^{er} dépôt de l'article textile.

Dans un mode de réalisation de l'invention, le moyen de mise à la terre auquel est connectée la 2^{ème} extrémité du fil de connexion peut consister en une tige métallique de mise à la terre qui est placée directement dans la terre (par exemple enfoncée dans le jardin ou au sous-sol d'une habitation).

Dans un autre mode de réalisation de l'invention, le moyen de mise à la terre auquel est connectée la 2^{ème} extrémité du fil de connexion peut consister en une prise électrique avec terre. Dans ce mode de réalisation, la 2^{ème} extrémité du fil de connexion peut comprendre un moyen de connexion configuré pour être branchée dans cette prise électrique avec terre.

En outre, afin d'éviter des chocs électriques, le fil de connexion peut comprendre avantageusement un fusible.

Lorsque l'article textile est une assise pour siège d'automobile, la 2^{ème} extrémité du fil de connexion peut être configurée pour être fixée à un moyen de mise à la terre qui consiste en un composé métallique de l'automobile qui sert de terre. Par exemple, ce moyen de mise à la terre peut être un élément métallique, de préférence positionné sous le siège, qui est connecté de manière conductrice au châssis de l'automobile. La 2^{ème} extrémité du fil de connexion peut comprendre un moyen de connexion consistant en une pince crocodile qui peut être fixée sur ledit élément métallique. L'utilisation de la masse (anode) de l'allume cigare peut être utilisée dans la mesure ou la cathode est shuntée.

Lorsque l'article textile selon l'invention est une couverture pour équidés, le système de mise à la terre consiste en les fers à cheval (à savoir les bandes de métal recourbées en « U » qui servent à protéger de l'usure le dessous des sabots des équidés). En effet, les fers à cheval permettent la mise à la terre de la couverture pour équidés selon l'invention de manière à ce que l'équidé bénéficie de tous les avantages détaillés ci-dessus qui sont procurés par ladite couverture. Dans ce mode de réalisation de l'invention, le 1^{er} dépôt n'est donc pas en contact avec le système de mise à la terre.

L'article textile peut être choisi dans le groupe constitué par les nattes de lit, les surmatelas, les draps, les couvertures, les assises de chaise ou siège, notamment des sièges d'automobile ou de bureau, les couvertures pour les équidés, les tapis, les tapis de yoga, les plaids, les coussins et les vêtements (par exemple les vestes doudoune).

Il peut s'agir, plus généralement, de tout article textile de toute forme appropriée pour permettre le contact d'une partie du corps avec le 1^{er} dépôt dudit article textile, et ce pour notamment un moment de détente de l'utilisateur afin d'obtenir tous les bienfaits sur la santé de cet article textile.

De préférence, il s'agit d'une couverture pour équidés.

L'invention concerne également un procédé de fabrication d'un article textile selon l'invention tel que décrit ci-dessus. Le procédé peut comprendre au moins les étapes suivantes :
a) on recouvre totalement ou en partie la 1^{ère} face de la couche de matériau textile avec le 1^{er} dépôt ;
b) on recouvre totalement ou en partie la 2^{ème} face de la couche de matériau textile avec le 2^{ème} dépôt.

En d'autres termes, à l'étape a), la 1^{ère} face de la couche de matériau textile est enduite totalement ou en partie avec ledit 1^{er} dépôt. A l'étape b), la 2^{ème} face de la couche de matériau textile est enduite totalement ou en partie avec ledit 2^{ème} dépôt.

De préférence, à l'étape a), on recouvre totalement la 1^{ère} face de la couche de matériau textile avec le 1^{er} dépôt.

Avantageusement, on recouvre totalement ou en partie à l'aide d'une racle la 1^{ère} face de la couche de matériau textile avec le 1^{er} dépôt. Ensuite, de manière préférée, on fait sécher au four ce 1^{er} dépôt sur la couche de matériau textile à une température comprise entre 100°C et 200°C, de préférence entre 115°C et 185°C, plus préférentiellement entre 130°C et 170°C, pendant une durée comprise entre 30 secondes et 10 minutes, de préférence entre 1 minute et 7 minutes, plus préférentiellement entre 2 minutes et 5 minutes.

De préférence à l'étape b), on recouvre totalement la 2^{ème} face de la couche de matériau textile avec le 2^{ème} dépôt.

Avantageusement, on recouvre totalement ou en partie à l'aide d'une racle la 2^{ème} face de la couche de matériau textile avec le 2^{ème} dépôt. Ensuite, de manière préférée, on fait sécher au four ce 2^{ème} dépôt sur la couche de matériau textile à une température comprise entre 100°C et 200°C, de préférence entre 115°C et 185°C, plus préférentiellement entre 130°C et 170°C, pendant une durée comprise entre 30 secondes et 10 minutes, de préférence entre 1 minute et 7 minutes, plus préférentiellement entre 2 minutes et 5 minutes.

Ainsi, le procédé de fabrication d'un article selon l'invention est simple à mettre en oeuvre. En effet, il met en oeuvre des étapes d'enduction sur chacune des faces de la couche de matériau textile avec respectivement ledit 1^{er} dépôt et ledit 2^{ème} dépôt. Ce procédé est peu onéreux et nécessite un minimum de matières. Il se distingue des procédés de fabrication de dispositifs de mise à la terre connu de l'art antérieur, du fait qu'il ne nécessite pas de superposer et d'assembler ensemble différentes couches de matériaux textiles, par exemple par laminage, collage ou au moyen de coutures.

L'invention sera mieux comprise à l'aide de la description détaillée qui est exposée ci-dessous en référence au dessin annexé représentant, à titre d'exemple non limitatif, deux articles textiles selon l'invention.

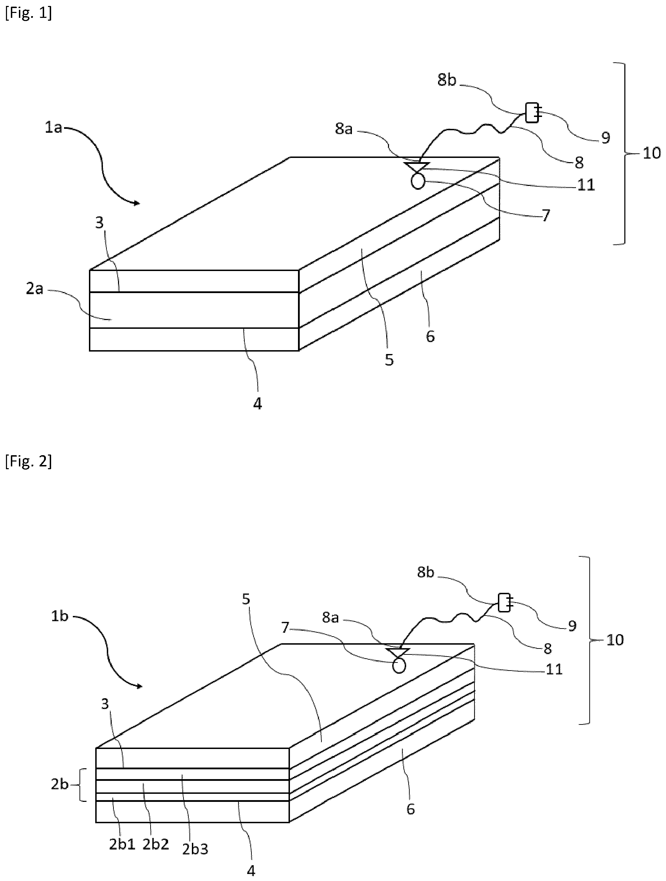

[Fig. 1] La figure 1 est une vue en perspective d'un 1^{er} article textile selon l'invention.

[Fig. 1] La figure 2 est une vue en perspective d'un 2^{ème} article textile selon l'invention.

Sur la figure 1 est représenté en perspective et de manière schématique un 1^{er} article textile 1a selon l'invention. Plus précisément, il s'agit d'une natte de lit de largeur 75 cm et de longueur 190 cm.

La natte 1a comprend :
- une couche d'un matériau textile 2a en coton présentant une 1^{ère} face 3 et une 2^{ème} face 4,
- un système de mise à la terre 10 de l'article textile 1a.

La 1^{ère} face 3 de la couche de matériau textile 2a est totalement recouverte d'un 1^{er} dépôt 5 d'une épaisseur de 200 µm et qui comprend, en pourcentages massiques exprimés par rapport à la masse du 1^{er} dépôt 5 : entre 90 et 98% de polyuréthane et d'agents réticulants, ainsi que 2 à 10 % de noir de carbone.

La 2^{ème} face 4 de la couche de matériau textile 2a est totalement recouverte d'un 2^{ème} dépôt 6 d'une épaisseur de 200 µm et qui comprend, en pourcentages massiques exprimés par rapport à la masse du 2^{ème} dépôt 6 : entre 50 et 95% de polyuréthane et d'agents réticulant, ainsi que 5 à 50% d'un mélange d'oxydes métalliques.

Le système de mise à la terre 10 de l'article textile 1a comprend :
- un fil de connexion 8 en cuivre qui comprend une 1^{ère} extrémité 8a et une 2^{ème} extrémité 8b,
- un élément de connexion 7 à la 1^{ère} extrémité 8a du fil de connexion 8 qui consiste en un rivet et que comprend le 1^{er} dépôt 5,
- la 1^{ère} extrémité 8a du fil de connexion 8 comprend une pince crocodile 11, ladite extrémité 8a du fil de connexion 8 est ainsi connectée au 1^{er} dépôt 5 lorsque la pince crocodile 11 est en contact avec le rivet 7,
- la 2^{ème} extrémité 8b du fil de connexion 8 comprend une prise électrique 9 configurée pour être branchée dans une prise de courant (non représentée sur la figure 1) reliée à la terre.

Sur la figure 2 est représenté en perspective et de manière schématique un 2^{ème} article textile 1b selon l'invention.

Le 2^{ème} article textile 1b comprend une couche d'un matériau textile 2b.

Le 2^{ème} article textile 1b diffère du 1^{er} article textile 1a uniquement en ce que la couche de matériau textile 2b se compose d'une 1^{ère} couche de polyester 2b1 d'un grammage de 110 g/cm² sur laquelle est disposée une 2^{ème} couche de matériau textile de rembourrage 2b2 comprenant de la ouate. Une 3^{ème} couche de matériau textile de polyester 2b3 de grammage 110 g/cm² est disposée sur ladite 2^{ème} couche de matériau textile 2b2. La couche de matériau textile 2b se décompose en 3 couches de matériau textile 2b1, 2b2, 2b3 qui sont superposées les unes sur les autres.

Dans l'article textile 1b :
- le 1^{er} dépôt 5 recouvre la 3^{ème} couche de matériau textile 2b3,
- le 2^{ème} dépôt 6 recouvre la 1^{ère} couche de matériau textile 2b1.

### PARTIE EXPERIMENTALE :

### 1^{ère} série d'expérimentations :

Une 1^{ère} série d'expérimentations a été réalisée sur des nattes de lit, des assises de siège de bureau et des couvertures pour équidés afin de confirmer les effets bénéfiques procurés par ces articles textiles selon l'invention.

### 1) Natte de lit :

30 sportifs ont dormi 15 jours sans natte et ensuite 3 semaines sur une natte de lit selon l'invention. Il a été constaté une augmentation permanente de la moyenne de leur VFC au fur et à mesure qu'ils utilisaient la natte.

La VFC est la variation dans le temps de battements cardiaques consécutifs. La VFC est censée correspondre à l'équilibre entre les influences sympathique et parasympathique sur le rythme intrinsèque du coeur. La VFC est liée au mode de vie, à l'activité physique, aux habitudes alimentaires, au rythme du sommeil et au tabagisme. La diminution de la VFC est associée aux risques d'événements cardiaques chez les adultes. Elle est un facteur prédictif de l'hypertension artérielle. Une VFC diminuée est liée à un risque accru de décès et elle pourrait avoir une valeur prédictive pour l'espérance de vie et la santé. Chez les sportifs la VFC est contrôlée régulièrement car une baisse de celle-ci témoigne d'un état de fatigue ou d'un surentrainement. Elle est le témoin direct de bons facteurs de récupération.

De plus, les sportifs se sont senti en meilleure forme et ont pu augmenter leur charge de travail.

Grâce à la natte de lit selon l'invention, le parasympathique a été plus fortement stimulé et la récupération a été maximisée. Les sportifs n'ont en général pas ou peu ressenti de courbatures le matin et beaucoup ont vu disparaître leurs crampes à l'effort.

### 2) Assise de bureau :

Des tests ont été réalisés sur 25 personnes travaillant 8 heures par jour devant un ordinateur en étant assises sur un siège de bureau comprenant une assise selon l'invention. On a noté une augmentation de leur vigilance et une baisse de leur fatigabilité au cours de la journée.

### 3) Couverture pour chevaux :

Des tests ont été réalisés avec des chevaux de courses de trot que l'on a équipés de couvertures pour équidés selon l'invention. Une meilleure récupération et une augmentation de la VFC ont été observées pour tous les chevaux. De plus, l'entraîneur a constaté que les chevaux étaient plus calmes, plus souples et plus enclins à reprendre l'entraînement.

### 2^{ème} série d'expérimentations :

Une 2^{ème} série expérimentations a été réalisée avec deux articles textiles selon l'invention, ainsi qu'avec un article textile comparatif.

Le 1^{er} article textile était une couche de coton (grammage 150 g/m²) destiné à la confection de surmatelas et de couvertures pour chevaux. La 1^{ère} face de cette couche de coton était totalement recouverte d'un 1^{er} dépôt comprenant un polyuréthane chargé en poudre de noir de carbone à hauteur de 10 g/m². La 2^{ème} face de cette couche de coton était totalement recouverte d'un 2^{ème} dépôt comprenant un polyuréthane chargé en oxydes métalliques à hauteur de 45 g/m².

Ce 1^{er} article textile a été mis à la terre grâce à un système de mise à la terre approprié et à un élément de connexion que comprenait le 1^{er} dépôt qui était relié audit système de mise à la terre.

Une fois le 1^{er} article textile branché à la terre, une tension de 250 à 500 millivolts en courant continu a été obtenue. Des sujets sportifs ont été mis en contact avec ce 1^{er} article textile et plus précisément au niveau du 1^{er} dépôt.

Il a été observé avec ce 1^{er} article textile un taux d'émissivité de 95% de rayons à des longueurs d'onde comprises entre 3 µm et 20 µm (c'est-à-dire dans le domaine de l'IR) avec une puissance radiative de 156 W / m².

Ces caractéristiques du 1^{er} article textile ont eu pour conséquence une augmentation de la VFC chez ces sujets sportifs. De plus, pour certains d'entre eux, leur temps de sommeil profond est passé de 15 minutes à 1 heure.

Le 2^{ème} article textile était composé des 3 couches suivantes qui étaient superposées les unes sur les autres :
- la couche du dessus de la pile était une couche de coton (grammage 120 g/m²) dont la face opposée à la face en contact avec la couche intermédiaire était recouverte d'un 1^{er} dépôt comprenant un polyuréthane chargé en poudre de noir de carbone à hauteur de 10 g/m²;
- la couche intermédiaire de la pile était une couche de ouate (grammage 120 g/m²) ;
- la couche inférieure de la pile était une couche de coton (grammage 120 g/m²) dont la face opposée à la face en contact avec la couche intermédiaire était recouverte d'un 2^{ème} dépôt comprenant un polyuréthane chargé en oxydes métalliques à hauteur de 45 g/m².

Ce 2^{ème} article textile était destiné à la confection de surmatelas rembourrés et de couvertures pour chevaux pour l'hiver.

Ce 2^{ème} article textile a été mis à la terre grâce à un système de mise à la terre approprié et à un élément de connexion que comprenait le 1^{er} dépôt qui était relié audit système de mise à la terre.

Une fois le 2^{ème} article textile branché à la terre, une tension de 250 à 500 millivolts en courant continu a été obtenue. Des sujets sportifs ont été mis en contact avec ce 2^{ème} article textile et plus précisément au niveau du 1^{er} dépôt.

Il a été observé avec ce 2^{ème} article textile un taux d'émissivité de 90% des rayons à des longueurs d'onde comprises entre 3 µm et 20 µm (c'est-à-dire dans le domaine de l'IR) avec une puissance radiative de 140 W / m².

Ces caractéristiques du 2^{ème} article textile ont eu pour conséquence une augmentation de la VFC chez ces sujets sportifs. De plus, pour certains d'entre eux, leur temps de sommeil profond est passé de 15 minutes à 1 heure.

Le 3^{ème} article textile était un article textile comparatif. Plus précisément, il s'agissait d'une natte de lit commercialisée en Chine qui comprenait les deux couches suivantes superposées l'une sur l'autre :
- une 1^{ère} couche d'un tissu tissé avec du fil de carbone incorporant quelques lignes de fils d'argent ;
- une 2^{ème} couche d'un tissu tissé avec des fils au sein desquels avait été incorporée de la tourmaline lors du procédé de fabrication desdits fils.

Les deux couches de tissus étaient contrecollées l'une sur l'autre et les bords de l'ensemble ainsi obtenu étaient cousus.

### La tourmaline est connue pour émettre des rayons IR.

Une fois le 3^{ème} article textile comparatif branché à la terre, une tension de 150 à 200 millivolts en courant continu a été obtenue. Des sujets sportifs ont été mis en contact avec ce 3^{ème} article textile comparatif et plus précisément au niveau de la 1^{ère} couche de tissu précitée.

Il a été observé avec ce 3^{ème} article textile comparatif un taux d'émissivité de seulement 80% des rayons à des longueurs d'onde comprises entre 3 µm et 12 µm (c'est-à-dire dans le domaine de l'IR) avec une puissance radiative de seulement 45 W / m².

De plus, il n'a pas été observé d'augmentation de la VFC avec le 3^{ème} article textile comparatif.

Au vu de ces résultats, on relève que les deux articles textiles selon l'invention sont bien plus efficaces que le 3^{ème} article textile comparatif en ce qui concerne le renvoi des rayons IR émis par le corps humain. De plus, lors de la mise à la terre des 2 articles textiles selon l'invention, leur 1^{er} dépôt a permis un meilleur passage des électrons de manière continue sur lesdits articles textiles selon l'invention pour qu'ils parcourent la surface du corps que le 3^{ème} article textile comparatif. Autrement dit, les 2 articles textiles selon l'invention reproduisent mieux le phénomène naturel de décharge électrostatique que le 3^{ème} article textile comparatif.

Ces meilleures performances des articles textiles selon l'invention s'expliquent par le fait que :
- le 1^{er} dépôt comprenant un mélange de polyuréthane et de poudre de noir de carbone permet de recouvrir la couche de matériau textile avec un grammage plus élevé en matériau conducteur que celui de la 1^{ère} couche du tissu tissé avec du fil de carbone incorporant quelques lignes de fils d'argent du 3^{ème} article textile comparatif ;
- le 2^{ème} dépôt comprenant un mélange de polyuréthane et d'oxydes métalliques permet de recouvrir la couche de matériau textile avec un grammage plus élevé en oxydes métalliques que celui de la 2^{ème} couche du tissu tissé avec des fils au sein desquels était présente de la tourmaline du 3^{ème} article textile comparatif.

La quantité plus importante de matériau conducteur dans le 1^{er} dépôt permet aux électrons de mieux remonter et parcourir la surface du corps. De plus, la quantité plus importante d'oxydes métalliques permet le renvoi de rayons IR émis par le corps de manière plus performante ; ce qui stimule mieux la microcirculation des muscles et des organes, et ainsi les électrons pénètrent beaucoup plus profondément sous le derme afin de venir directement au coeur des zones d'oxydation.

En d'autres termes, les quantités plus importantes en matériau conducteur et en oxydes métalliques dans les articles textiles selon l'invention grâce respectivement aux 1^{er} et 2^{ème} dépôts leur procurent de meilleures performances que celles du 3^{ème} article textile comparatif.

## Revendications

1. Article textile (1a,1b) configuré pour être mis à la terre qui comprend au moins :
- une couche d'un matériau textile (2a,2b,2b1,2b2,2b3) présentant une 1^{ère} face (3) et une 2^{ème} face (4),
- un système de mise à la terre (10) de l'article textile (1a,1b),
**caractérisé en ce que** :
- la 1^{ère} face (3) de la couche de matériau textile (2a,2b,2b1,2b2,2b3) est totalement ou en partie recouverte d'un 1^{er} dépôt (5) comprenant au moins un polymère et des particules d'au moins un matériau conducteur, ledit 1^{er} dépôt (5) étant optionnellement en contact avec le système de mise à la terre (10),
- la 2^{ème} face (4) de la couche de matériau textile (2a,2b,2b1,2b2,2b3) est totalement ou en partie recouverte d'un 2^{ème} dépôt (6) comprenant au moins un polymère et des oxydes métalliques.

2. Article textile (1a,1b) selon la revendication 1, **caractérisé en ce que** l'épaisseur du 1^{er} dépôt (5) est comprise entre 20 µm et 2 mm, de préférence entre 100 µm et 1 mm.

3. Article textile (1a,1b) selon la revendication 1 ou 2, **caractérisé en ce que** l'épaisseur du 2^{ème} dépôt (6) est comprise entre 20 µm et 2 mm, de préférence entre 100 µm et 1 mm.

4. Article textile (1a,1b) selon l'une quelconque des revendications 1 à 3, **caractérisé en ce que** le matériau textile est choisi parmi le coton, le polyamide, le polyester, la viscose, le polypropylène, des matériaux ignifugés, la laine et la soie.

5. Article textile (1a,1b) selon l'une quelconque des revendications 1 à 4, **caractérisé en ce que** l'épaisseur de la couche de matériau textile (2a,2b,2b1,2b2,2b3) est comprise entre 1 mm et 5 cm.

6. Article textile (1b) selon l'une quelconque des revendications 1 à 5, **caractérisé en ce que** la couche de matériau textile (2b) est composée de plusieurs couches de matériau textile (2b1,2b2,2b3) qui sont superposées les unes sur les autres.

7. Article textile (1a,1b) selon l'une quelconque des revendications 1 à 6, **caractérisé en ce que** le polymère du 1^{er} dépôt (5) est choisi dans le groupe constitué par les acryliques, les polyuréthanes et les silicones, pris seul ou en combinaison de ceux-ci.

8. Article textile (1a,1b) selon l'une quelconque des revendications 1 à 7, **caractérisé en ce que** le 1^{er} dépôt (5) comprend, en pourcentages massiques exprimés par rapport à la masse du 1^{er} dépôt :
- entre 1% et 10% de particules de matériau conducteur, de préférence de noir de carbone,
- entre 90% et 99% de polymère.

9. Article textile (1a,1b) selon l'une quelconque des revendications 1 à 8, **caractérisé en ce que** le polymère du 2^{ème} dépôt (6) est choisi dans le groupe constitué par les acryliques, les polyuréthanes, les copolymères polystyrène/butadiène, le latex naturel ou synthétique, le gel d'élastomère de silicone, l'élastomère de silicone, le caoutchouc butyle, le caoutchouc naturel, le caoutchouc nitrile, le styrène bloc copolymère et le styrène, pris seul ou en combinaison de ceux-ci.

10. Article textile (1a,1b) selon l'une quelconque des revendications 1 à 9, **caractérisé en ce que** les oxydes métalliques du 2^{ème} dépôt (6) sont choisis dans le groupe constitué par les oxydes d'aluminium, de titane, de zirconium, d'yttrium, de magnésium, de silicium, de fer, de cobalt, de manganèse, de cuivre, de zinc et de chrome, pris seul ou en combinaison de ceux-ci.

11. Article textile (1a,1b) selon l'une quelconque des revendications 1 à 10, **caractérisé en ce que** le 2^{ème} dépôt (6) comprend, en pourcentages massiques exprimés par rapport à la masse du 2^{ème} dépôt (6) :
- entre 5% et 50% d'oxydes métalliques,
- entre 50% et 95% de polymère,
- optionnellement entre 5% et 20% de solvant,
- optionnellement entre 2% et 20% d'un composé hydrophile choisi parmi les carboxy-méthyl-celluloses et le polyéthylène glycol,
- optionnellement entre 1% et 10% de composés additionnels.

12. Article textile (1a,1b) selon l'une quelconque des revendications 1 à 11, **caractérisé en ce qu'**il est choisi dans le groupe constitué par les nattes de lit, les surmatelas, les draps, les couvertures, les assises de chaise ou siège, les couvertures pour les équidés, les tapis, les tapis de yoga, les plaids, les coussins et les vêtements.

13. Procédé de fabrication d'un article textile (1a,1b) selon l'une quelconque des revendications 1 à 12, **caractérisé en ce qu'**il comprend au moins les étapes suivantes :
a) on recouvre totalement ou en partie la 1^{ère} face (3) de la couche de matériau textile (2a,2b,2b1,2b2,2b3) avec le 1^{er} dépôt ;
b) on recouvre totalement ou en partie la 2^{ème} face (4) de la couche de matériau textile (2a,2b,2b1,2b2,2b3) avec le 2^{ème} dépôt (6).

14. Procédé de fabrication d'un article textile (1a,1b) selon la revendication 13, **caractérisé en ce qu'**on recouvre totalement ou en partie à l'aide d'une racle la 1^{ère} face (3) de la couche de matériau textile (2a,2b,2b1,2b2,2b3) avec le 1^{er} dépôt (5), puis on fait sécher au four ce 1^{er} dépôt (5) sur la couche de matériau textile (2a,2b,2b1,2b2,2b3) à une température comprise entre 100°C et 200°C pendant une durée comprise entre 30 secondes et 10 minutes.

15. Procédé de fabrication d'un article textile (1a,1b) selon la revendication 13 ou 14, **caractérisé en ce qu'**on recouvre totalement ou en partie à l'aide d'une racle la 2^{ème} face (4) de la couche de matériau textile avec le 2^{ème} dépôt (6), puis on fait sécher au four ce 2^{ème} dépôt (6) sur la couche de matériau textile (2a,2b,2b1,2b2,2b3) à une température comprise entre 100°C et 200°C pendant une durée comprise entre 30 secondes et 10 minutes.
